# EUROPEAN PATENT APPLICATION

(11) **EP 0 752 586 A1**
(43) Date of publication of application: **08.01.1997**
(21) Application number: 95913328.1
(22) Date of filing: 23.03.1995
(51) Int. Cl.: G01N 33/569, G01N 33/545

(54) **ANTIBODY-SENSITIZED LATEX USED FOR DETECTING NITRATE OR NITRITE BACTERIA**

(30) Priority: 24.03.1994 JP 76336/94
(71) Applicant: KABUSHIKI KAISHA YAKULT HONSHA, Minato-ku Tokyo 105 (JP)
(72) Inventor: ATOBE, Hisao, Tachikawa-shi, Tokyo 190 (JP); YAGISHITA, Masami, Nagareyama-shi, Chiba 270-01 (JP); OHMURA, Hiroshi, Kabushiki Kaisha Yakult Honsha, Tokyo 105 (JP); NAGAI, Fumiko, Kabushiki Kaisha Yakult Honsha, Tokyo 105 (JP)
(74) Representative: Thomsen, Dieter, Dr.
(86) International application number: JP9500539
(87) International publication number: WO9525958

(57) **Abstract**

The object of the present invention is to provide an antibody-sensitized latex, which is used in a method for detecting conveniently nitrate or nitrite bacteria present in activated sludge or soil. The present invention relates to an antibody-sensitized latex used for detecting nitrate or nitrite bacteria, comprising latex particles and an antibody, wherein the antibody recognizes nitrate or nitrite bacteria specifically, and is adsorbed on the latex particles. As the latex particles, the latex particles having a high specific gravity of about 1.5 g/cc and an average particle diameter of about 1.0 µm are used. In a conventional method for detecting nitrate or nitrite bacteria, a long period of time and technical expertise have been required, however, according to the present invention, the detection of nitrate or nitrite bacteria can be performed easily and conveniently. Further, it serves to establish a method for controlling the number of the active bacterium present in activated sludge or soil rapidly and properly.

## Description

### Technical Field

The present invention relates to an antibody-sensitized latex used for facilitating the detection of nitrate or nitrite bacteria being present in activated sludge or soil; more specifically, the present invention relates to a novel antibody-sensitized latex, which facilitates the detection of nitrate or nitrite bacteria which has taken a long period of time or has required technical expertise, and further serves to establish a method for controlling rapidly and properly the number of targeted active bacterium being present in activated sludge or soil.

### Background of Arts

As a method for detecting nitrate or nitrite bacteria being present in activated sludge or soil has been known a method for measuring the bacteria indirectly according to the presence of nitrite produced in a sample of the activated sludge or soil collected, after culturing the bacteria for the period of one to two months (MPN method, and the like).

Usually, the targeted bacterium having an activity (ammonia oxidation, nitrite oxidation) being present in activated sludge or soil has been controlled by utilizing said method.

On the other hand, the present inventors have engaged in assiduous studies on a method for detecting rapidly nitrate or nitrite bacteria, and have already reported that such an object can be accomplished by an enzyme antibody method using an antibody which recognizes specifically nitrate or nitrite bacteria (Official Gazette of Japanese Laid-Open Patent Publication No. 5-322896).

In the above invention, the present inventors have confirmed that it is possible to detect nitrate or nitrite bacteria by an enzyme antibody method using an antibody which recognizes specifically nitrate or nitrite bacteria; however said method requires technical expertise, and it has been demanded to establish a simplified method, and therefore the present inventors have further made assiduous studies with a view to developing a method for detecting nitrate or nitrite bacteria more conveniently.

As a result, the present inventors have found that nitrate or nitrite bacteria can be detected more conveniently by employing an antibody-sensitized latex comprising a latex particles and an antibody specific for nitrate or nitrite bacteria and being adsorbed on latex particles, which has led to the accomplishment of the present invention.

### Summary of the Invention

The object of the present invention is to provide an antibody-sensitized latex, which is used in a method for detecting conveniently nitrate or nitrite bacteria present in activated sludge or soil.

The present invention relates to an antibody-sensitized latex used for detecting nitrate or nitrite bacteria, comprising latex particles and an antibody, wherein the antibody recognizes nitrate or nitrite bacteria specifically, and is adsorbed on the latex particles. As the latex particles, the latex particles having a high specific gravity of about 1.5 g/cc and an average particle diameter of about 1.0 µm are used.

In a conventional method for detecting nitrate or nitrite bacteria, a long period of time and technical expertise have been required, however, according to the present invention, the detection of nitrate or nitrite bacteria can be performed easily and conveniently. Further, it serves to establish a method for controlling the number of the active bacterium present in activated sludge or soil rapidly and properly.

### Disclosure of the Invention

The object of the present invention is to provide a novel antibody-sensitized latex used for facilitating the detection of nitrate or nitrite bacteria present in activated sludge or soil.

It is another object of the present invention to establish a method for controlling the number of targeted bacterium having an activity (ammonia oxidation, nitrite oxidation) being present in activated sludge or soil rapidly and properly by utilizing the above antibody-sensitized latex.

The antibody-sensitized latex used for the detection of nitrate or nitrite bacteria of the present invention is characterized by comprising latex particles and an antibody, wherein the antibody is specific for nitrate or nitrite bacteria, namely, the antibody recognizes these bacteria specifically, and is adsorbed on the latex particles.

Besides, the present invention is characterized by employing latex particles having a high specific gravity of around 1.5 g/cc.

Besides, the present invention is characterized by employing latex particles having an average particle diameter of around 1.0 µm.

In the present invention, Nitrobacter agilis, IFO14297 strain, and Nitrosomonas europaea, IFO14298 strain, were used as standard strains of nitrate and nitrite bacteria for preparing antibodies; however, it goes without saying that antibodies can be prepared similarly by using other strains of nitrate and nitrite bacteria. As the antibodies specific for these bacteria are used an antiserum prepared according to a known method (e.g., method described in the Official Gazette of Japanese Laid-Open Patent Publication No. 5-322896 and the like) and purified with a suitable means for purification such as the protein A column kit.

Since the antibodies specific for these bacteria react with general nitrate and nitrite bacteria specifically, they can be utilized for the detection of general nitrate and nitrite bacteria (hereinafter may be referred to as nitrifying bacteria).

That is, in order to prepare the antibody specific for the above nitrifying bacteria, first of all, the bacterium are cultured with a suitable liquid medium, sterilized if necessary, and collected to obtain an antigen. Subsequently, the antigen is injected into the vein of a rabbit, and after confirmed the increase of the antibody titer observed, blood is collected from it and is subjected to a suitable treatment such as centrifugal separation to obtain antiserum.

The antibody is prepared by purifying it with a suitable means for purification such as the protein A column kit if necessary.

Subsequently, as latex particles of a carrier to be adsorbed by the above specific antibody, those having a specific gravity of about 1.0 to 1.5 g/cc, preferably around 1.5 g/cc and an average particle diameter of about 0.8 to 2.2 µm, preferably around 1.0 um can be utilized suitably; for example, commercially available products such as Bactlatex 0.81 (manufactured by Difco, average particle diameter: 0.81 µm, specific gravity: 1.0 g/cc), and H0901, H0902 and H2002 (manufactured by Nihon Gousei Gomu Co., Ltd., average particle diameter/specific gravity: 0.94 µm/1.5 g/cc, 0.98 µ m/1.5 g/cc and 2.22 µm/1.5 g/cc, respectively) can be utilized.

It has been revealed according to the examination by the present inventors, as described later, that in the case of those sensitized to latex particles having a specific gravity of around 1.0 g/cc, agglutination images can be judged sufficiently according to a method for observing them with a microscope (microscope latex method), but that it is difficult to judge agglutination images according to a method for observing them with the naked eye (microtiter method).

On the other hand, in those sensitized to latex particles having a high specific gravity of around 1.5 g/cc, latex particles agglutinated precipitate rapidly and can be judged with the naked eye easily and precisely; hence latex particles having a high specific gravity of around 1.5 g/cc are used preferably in the present invention.

As described later, a sensitized latex having a high sensitivity can be obtained by employing latex particles having an average particle diameter of around 1.0 µm; hence latex particles having an average particle diameter of around 1.0 µm are used preferably in the present invention.

The sensitized latex was prepared by mixing a suspension of latex particles diluted with a proper buffer and a solution of an antibody, leaving the mixture as it is for a while and then washing it.

As a buffer can be utilized an ammonium chloride buffered-saline solution, a PBS buffer and a glycine buffered-saline solution. Particularly, the glycine buffered-saline solution is used preferably from the viewpoints of properties of the agglutination and the difficulty in causing self-agglutination of a sensitized latex.

In this case, when the concentration of latex particles is adjusted from 0.05 to 1.0 %, preferably from 0.1 to 0.5 %, and more preferably around 0.25 %, then agglutination images formed can be easily observed.

The concentration of the antibody protein of an antibody solution to be sensitized, in the case of nitrate bacteria, is preferably from 0.03 to 0.3 mg/ml; when it is lower than 0.02 mg/ml, sensitivity of the latex deteriorates, and when it exceeds 0.4 mg/ml, it tends to be difficult to distinguish between positivity and negativity of it.

In the case of nitrite bacteria, it is preferably to adjust the concentration of it from 0.05 to 0.4 mg/ml; when it is lower than 0.04 mg/ml, sensitivity of the latex deteriorates, and when it exceeds 0.5 mg/ml, it tends to be difficult to distinguish between positivity and negativity of it.

A latex is sensitized at a temperature within the range of 0 to 60 °C ; and, if the latex is sensitized at room temperature or at a temperature slightly higher than it (to 40 °C ), a sensitized latex with a high sensitivity can be obtained.

The nitrate or nitrite bacteria is detected and the number of bacterium are measured by using such sensitized latices, mixing a sample liquid diluted to a proper grade and a latex solution and confirming the degree of dilution capable of observing agglutination images, on the other hand, performing a mixing test employing a standard sample of a bacterium liquid, and calculating the number of bacterium in comparison with the result of it.

As a specific method in the present invention can be employed the microtiter method capable of judging the agglutination images with the naked eye simply. That is, they can be observed and judged with the naked eye or with a magnifier of 10 magnifications by sampling an antigen or sample solutions diluted gradually with a buffer onto a U-shaped microplate, pouring an antibody-sensitized latex into each hole of the plate and leaving it as it is at room temperature for 3 to 15 hours. Specifically, agglutination images of each hole are judged by deeming agglutination images in the case of a buffer alone as negative, and the number of bacterium are calculated from the rate of dilution of the hole to be tested showing positive agglutination images and the result of an agglutination test employing a standard antigen.

It goes without saying that, in the present invention, in addition to the above, a method for judging the presence of agglutination images with a light microscope by mixing an antibody-sensitized latex according to the present invention and a sample to be tested on a slide glass (microscope latex method) can be employed. The existence of latex particles as a carrier capable of adsorbing an antibody has been known; however, the actual use of it for the detection of nitrate and nitrite bacteria has not been reported, and the efficiency of it has been investigated by the present inventors for the first time; in particular, the fact that it is possible to establish a method for controlling the number of bacteria of targeted active bacterium being present in activated sludge or soil rapidly and properly has been found by the present inventors for the first time.

Moreover, generally, latex particles are not always effective to all antibodies, and these findings have been obtained only after the experimental investigation of the suitability and efficiency of an antibody specific for nitrifying bacteria and latex particles.

### Best Mode for Carrying out the Invention

Hereunder, the present invention will be described in more detail according to Example, but the present invention is to be deemed to being not restricted to said Example.

### Example

### 1. Preparation of a Bacterial Cells Solution used for an Antigen

### (1) Preparation of an Antigen Solution of Nitrite Bacteria

With a medium (medium 400 ml, medium composition: 0.5 g of ammonium sulfate, 0.3 g of sodium chloride, 1 g of dipotassium hydrogenphosphate, 0.3 g of magnesium sulfate heptahydrate, 0.03 g of ferrous sulfate heptahydrate and 7.5 g of calcium carbonate dissolved in 1 liter of distilled water), a nitrite bacteria, Nitrosomonas europaea IFO14298 was cultured in a Sakaguchi flask (2 liters) under shaking condition. The quantitative analysis of nitrate formed was performed with the passage of time employing a Griess-Ilosvay reagent; the culture was terminated at the time when the growth of bacteria became highest, and into the flask, citric acid powder was added to adjust a pH to 3.5 and to dissolve non-dissolved calcium carbonate; and the reaction mixture was centrifuged to condense it, and into the resultant solution was added an equivalent amount of a 0.6 % formalin solution to sterilize it, and the resultant solution was washed to obtain an antigen solution.

### (2) Preparation of an Antigen Solution of Nitrate Bacteria

With a medium (medium composition: 1 g of sodium nitrite, 0.3 g of sodium chloride, 0.5 g of dipotassium hydrogen phosphate, 0.5 g of magnesium sulfate heptahydrate, 0.002 g of manganese sulfate hydrate and 0.005 g of ferric sulfate dissolved in 1 liter of distilled water), a nitrate bacterium, Nitrobacter agilis IFO14297 was cultured in a 1-liter jar fermenter by controlling pH of the medium to 7.5 with sodium hydroxide, assaying nitrite formed with the passage of time and adding sodium nitrite to the medium successively.

The cells suspension were centrifuged to condense them, and to the supernatant an equivalent amount of a 0.6 % formalin solution was added to sterilize it, and the resultant solution was washed to obtain an antigen solution.

### 2. Preparation of Corresponding Antiserum of Rabbit

Employing two KBL:Jw (Japanese white) rabbits, the antigen solution of nitrite bacteria and nitrate bacteria prepared by adjusting their concentration with turbidity (OD₆₆₀ ≒ 0.5) was injected in an amount of 0.5 ml into the vein of each. The antigen solution was injected additionally into it from 4 to 8 times every 7 to 10 days; from the third time on, an antibody titer was measured according to an enzyme antibody method on demand, and when an increase of the antibody titer was not observed, blood was collected from the heart of them. The blood was centrifuged to collect serum, and inactivated at 56 °C to obtain antiserum corresponding to nitrite and nitrate bacteria.

### 3. Preparation of an Antibody-Sensitized Latex

The thus prepared antiserum was purified with the protein A column kit (purchased from Amersham) and then diluted with a 0.1 M glycine buffered-saline solution (pH: 8.2) to adjust the protein concentration to 0.08 to 0.2 mg/l.

Into 1-volume of the antiserum was added 1-volume of a latex solution (manufactured by Nihon Gousei Gomu Co., Ltd., particle diameter: 0.98 µm, specific gravity: 1.5 g/cc); the reaction solution was mixed well and left as it is at 37 °C for one hour to allow the latex to adsorb the antibody. Then, into the solution was added a buffer containing 1 % BSA to terminate the reaction. The resultant solution was centrifuged to remove the surplus antibody and suspended in a liquid to adjust the latex concentration to 0.25 % and to store it.

### 4. Agglutination Reaction Test

The number of nitrite and nitrate bacteria was measured by the microtiter method and the microscope latex method employing a sensitized latex antibody according to the present invention.

That is, as a method for measuring the number of bacteria was employed the microscope latex method which comprises mixing the sensitized latex antibody and a sample to be tested on a slide glass, and observing and judging the presence of agglutination images with a light microscope, in addition to the above-described microtiter method.

As a comparative control test, a hemocytometer was used, and the value obtained by measuring the number of bacteria with a light microscope, which floating in a buffer in a sample to be tested, was deemed to be a standard value, and the value obtained by the MPN method (culture period: 40 to 60 days) according to 10 grades of dilution rate of from 10⁰ to 10⁹ employing 5 test tubes per group was deemed to be the value of the comparative control group.

### 5. Examination of Sensitization Conditions

### (1) Examination of the Optimum Temperature

Altering the temperature in sensitization of a latex to 56 °C , 37 °C , room temperature and 4 °C , the influence of the temperature on the detection of nitrifying bacteria was examined. Regarding other conditions, the latex concentration was adjusted to 0.5 %, a glycine buffered-saline solution was employed, and the antibody protein concentration was adjusted to 0.20 mg/ml in nitrite bacteria and 0.30 mg/ml in nitrate bacteria.

The results obtained are shown in Table 1. As is apparent from Table 1, it was revealed that the antibody-sensitized latex obtained by being sensitized at 37 °C had the highest sensitivity in both cases of nitrate bacteria and nitrite bacteria.

**Table 1**

| Influence of Temperature in the Sensitization on the Detection of Nitrifying Bacteria | | | | |
|---|---|---|---|---|
| Nitrifying bacterium | Temperature in the sensitization | | | |
| | 56°C | 37°C | Room temperature | 4°C |
| Nitrosomonas europaea | 1.2 x 10⁶ | 3.0 x 10⁵ | 1.2 x 10⁶ | 1.2 x 10⁶ |
| Nitrobacter agilis | 1.0 x 10⁶ | 5.0 x 10⁵ | 1.0 x 10⁶ | 1.0 x 10⁶ |
| (Numerical values in the table show the lower limit of the number of bacteria detected.) | | | | |

### (2) Examination of a Buffer

As a buffer to be used in sensitization of a latex, the following five kinds of buffers were prepared and the influence of each buffer in the sensitization on the detection of nitrifying bacteria was examined.
(i) 0.1M ammonium chloride buffered-saline solution (pH: 8.2)
(ii) 0.2M ammonium chloride buffered-saline solution (pH: 8.2)
(iii) PBS buffer (pH: 7.2)
(iv) 0.1M glycine buffered-saline solution (pH: 8.2)
(v) 0.01M glycine buffered-saline solution (pH: 8.2)

Regarding other conditions, the temperature of the sensitization was adjusted to 37 °C , the latex concentration was adjusted to 0.5 %, and the concentration of the antibody protein was adjusted to 0.20 mg/ml in nitrite bacteria and 0.30 mg/ml in nitrate bacteria.

The results obtained are shown in Table 2. As is apparent from Table 2, it was revealed that the antibody-sensitized latex obtained by employing the 0.01M glycine buffered-saline solution (pH: 8.2) had the highest sensitivity in both cases of nitrate bacteria and nitrite bacteria and did not cause self-agglutination.

**Table 2**

| Influence of Buffers in the Sensitization on the Detection of Nitrifying Bacteria | | | | | |
|---|---|---|---|---|---|
| Nitrifying bacterium | Buffer | | | | |
| | (i) | (ii) | (iii) | (iv) | (v) |
| Nitrosomonas europaea | 6.0 x 10⁵ | 6.0 x 10⁵ | 6.0 x 10⁵ | 3.0 x 10⁵ | 1.5 x 10⁵ |
| Nitrobacter agilis | 5.0 x 10⁵ | 5.0 x 10⁵ | 1.0 x 10⁶ | 5.0 x 10⁵ | 1.3 x 10⁵ |
| (Numerical values in the table show the lower limit of the number of bacteria detected.) | | | | | |

### (3) Examination of the Optimum Concentration of a Latex

The influence of the latex concentration on the detection of nitrifying bacteria was examined, altering the latex concentration in sensitization of the latex to 0.10 %, 0.25 % and 0.50 %. Regarding other conditions, the temperature of the sensitization was adjusted to 37 °C and the time of it was adjusted to one hour, employing a glycine buffered-saline solution, and the concentration of the antibody protein was adjusted to 0.20 mg/ml in nitrite bacteria and 0.30 mg/ml in nitrate bacteria.

The results obtained are shown in Table 3. As is apparent from Table 3, it was revealed that sensitivity of the latex tends to decrease at 0.10 % in both cases of nitrate bacteria and nitrite bacteria, and that 0.25 % is preferable from the viewpoints of the rate of precipitation and the simplicity in observing agglutination images.

**Table 3**

| Influence of the Latex Concentration on the Detection of Nitrifying Bacteria | | | |
|---|---|---|---|
| Nitrifying bacterium | Latex Concentration | | |
| | 0.50 % | 0.25 % | 0.10 % |
| Nitrosomonas europaea | 3.0 x 10⁵ | 3.0 x 10⁵ | 6.0 x 10⁵ |
| Nitrobacter agilis | 5.0 x 10⁵ | 5.0 x 10⁵ | 1.0 x 10⁶ |
| (Numerical values in the table show the lower limit of the number of bacteria detected.) | | | |

### (4) Examination of the Optimum Concentration of Antibody Protein in Sensitization of a Latex

The optimum concentration of antibody protein in sensitization of a latex was examined.

The optimum concentration was examined in the case of nitrite bacteria at 0.40, 0.30, 0.20, 0.15, 0.10, 0.08, 0.05 and 0.03 (mg/ml), and in the case of nitrate bacteria at 0.50, 0.40, 0.25, 0.20, 0.13, 0.10, 0.06 and 0.50 (mg/ml), respectively.

Regarding other conditions, the temperature of the sensitization was adjusted to 37 °C , the time of it was adjusted to one hour and the concentration of latex was adjusted to 0.5 %; a glycine buffered-saline solution was employed.

The results are shown in Table 4.

**Table 4**

| Influence of the Concentration of Antibody Protein in the Sensitization on the Detection of Nitrifying Bacteria | | | | | |
|---|---|---|---|---|---|
| Nitrosomonas europaea | Antibody protein concentration (mg/ml) | 0.40 | 0.30 | 0.20 | 0.15 |
| | The lower limit of the Number of bacterium detected | Unclear | Unclear | 3.0x10⁵ | 3.0x10⁵ |
| | | 0.10 | 0.08 | 0.05 | 0.03 |
| | | 3.0x10⁵ | 3.0x10⁵ | 1.2x10⁶ | 1.2x10⁶ |
| | | | | | |
| Nitrobacter agilis | Antibody protein concentration (mg/ml) | 0.50 | 0.40 | 0.25 | 0.20 |
| | The lower limit of the Number of bacterium detected | Unclear | Unclear | 5.0x10⁵ | 5.0x10⁵ |
| | | 0.13 | 0.10 | 0.06 | 0.05 |
| | | 5.0x10⁵ | 5.0x10⁵ | 1.0x10⁶ | 1.0x10⁶ |

As is apparent from Table 4, in the case of nitrite bacteria, when a latex was sensitized at a concentration lower than 0.05 mg/ml, sensitivity of the latex tended to decrease. On the other hand, when a latex was sensitized at a concentration higher than 0.30 mg/ml, the distinction between negativity and positivity of a reaction tended to be unclear. It was revealed that when a latex was sensitized at 0.20, 0.15, 0.10 and 0.08 (mg/ml), the bacterium targeted can be detected clearly.

In the case of nitrate bacteria, when a latex was sensitized at a concentration lower than 0.06 mg/ml, sensitivity of the latex tended to decrease. On the other hand, when a latex was sensitized at a concentration higher than 0.40 mg/ml, the distinction between negativity and positivity of a reaction tended to be unclear. When a latex was sensitized at 0.25 to 0.10 (mg/ml), the lower limit of the detection was almost same, but it was revealed that when a latex sensitized at 0.20, 0.13 and 0.10 (mg/ml) in particular, sensitivity of the latex is excellent.

### (5) Examination of Various Latex Particles

Latex particles to be sensitized by an antibody were examined.

Regarding latex particles, Bactlatex 0.81 (manufactured by Difco, average particle diameter: 0.81 µm, specific gravity: 1.0 g/cc) and Latex H0901, Latex H0902 and Latex H2002 (manufactured by Nihon Gousei Gomu Co., Ltd., average particle diameter/specific gravity: 0.94 µm/1.5 g/cc, 0.98 µm/1.5 g/cc and 2.22 µm/1.5 g/cc, respectively) were examined.

Regarding other conditions, under the condition of that the temperature of the sensitization was 37 °C , the time of it was one hour and the concentration of latex was 0.25 %, a latex was sensitized employing a 0.01M glycine buffered-saline solution.

The results obtained are shown in Table 5. As is apparent from Table 5, in the cases of latex particles having a specific gravity of around 1.0 and latex particles having an average particle diameter of around 2.0 were unclear the results obtained by the microtiter method by observing agglutination images with the naked eye, and it was needed to observe them with the microscope latex method; and, it was revealed that in the case of a latex with a high specific gravity of around 1.5, agglutination images can be observed clearly. In addition, it was revealed that those having an average particle diameter of around 1.0 µm can be used preferably in the microtiter method.

**Table 5**

| Examination of Various Latices for the Detection of Nitrifying Bacteria | | | | |
|---|---|---|---|---|
| Nitrifying bacterium | Latex | | | |
| | H0901 | H0902 | H2002 | Bactlatex 0.81 |
| Nitrosomonas europaea | 6.0 x 10⁵ | 3.0 x 10⁵ | Unclear | Unclear |
| Nitrobacter agilis | 5.0 x 10⁵ | 2.5 x 10⁵ | Unclear | Unclear |
| (Numerical values in the table show the lower limit of the number of bacteria detected.) | | | | |

### 6. Comparative Test between the Official Method and the Method of Measurement with a Microscope Employing Nitrifying Bacteria and Samples

In order to confirm the efficiency of a simplified method of measurement (microtiter method) with the latex having a high specific gravity according to the present invention, the results of a method by observing and determining an agglutination reaction with a microscope (microscope latex method) and the results of the MPN method of the official method were compared.

As samples were employed nitrite bacteria, nitrate bacteria used for the preparation of antibodies, and actual samples of activated sludge and soil. Samples No. 1 to No. 6 shown in Table 6 (Table 6-1 and Table 6-2) are test samples obtained by diluting a solution of bacteria used for the preparation of antibodies. The number of bacterium to be compared were counted with a microscope by means of a modified Neubauer, and the propriety of numerical values was examined in comparison with the number obtained above as the standard value.

The results obtained are shown in Table 7. As is apparent from Table 6, it was confirmed that in the cases of nitrite bacteria and nitrate bacteria used for the preparation of antibodies, the number of bacterium obtained by the microtiter method, the microscope latex method and the MPN method were consistent with the standard number.

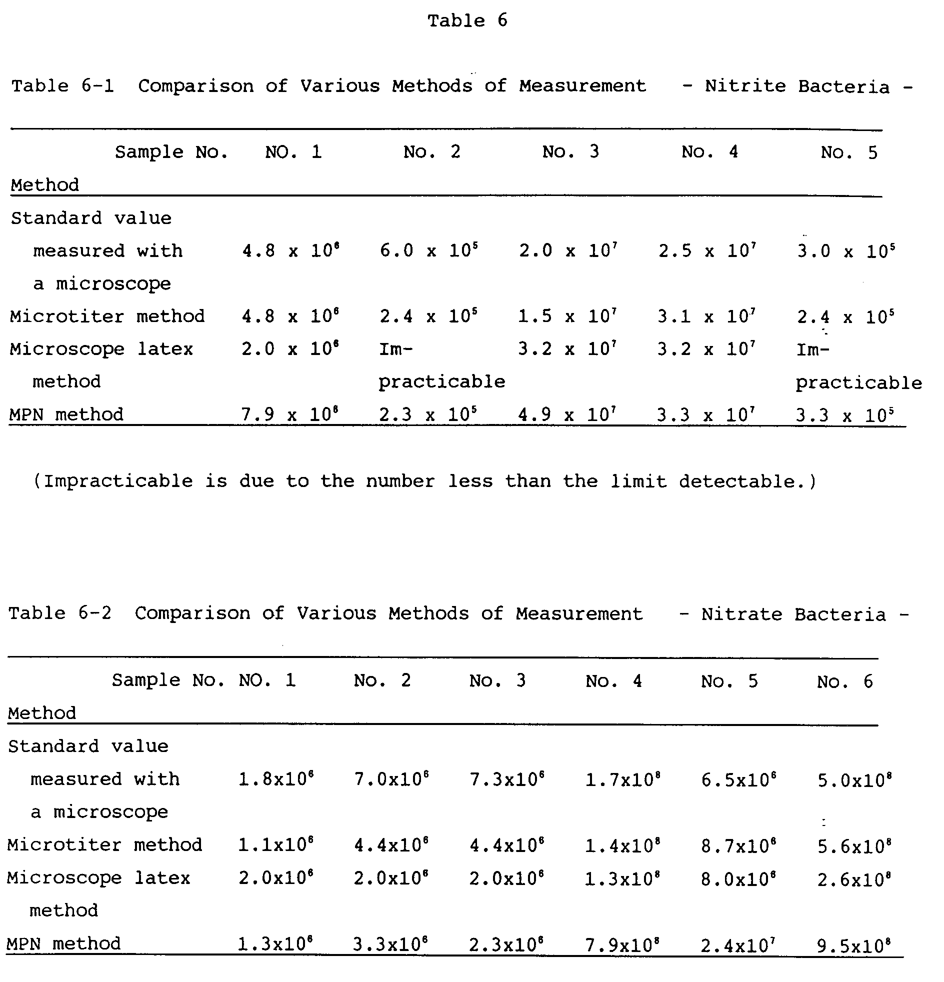

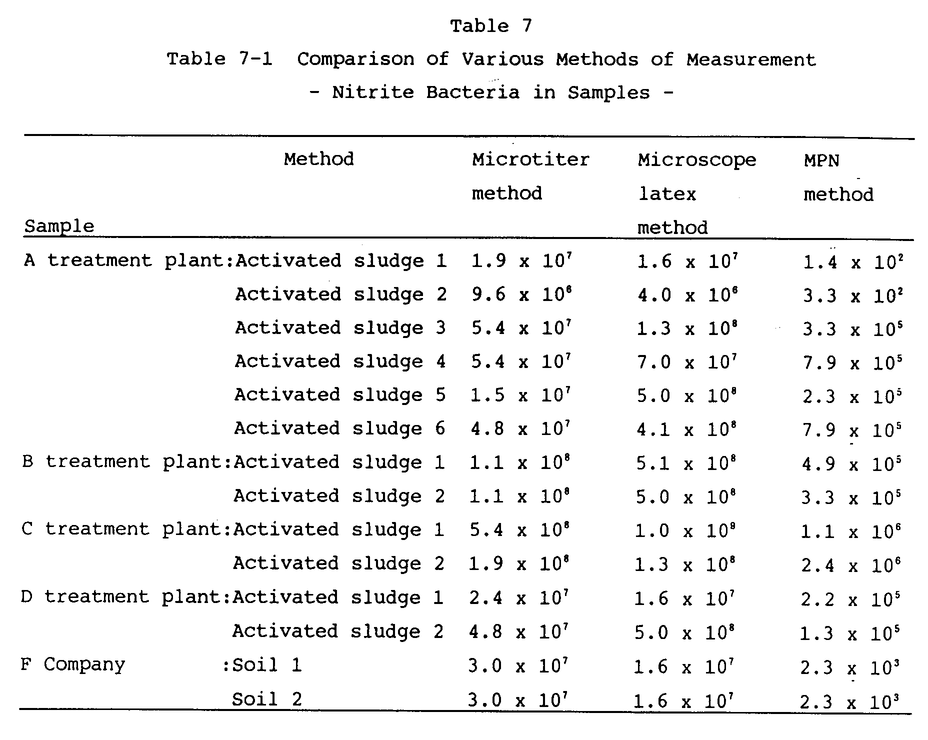

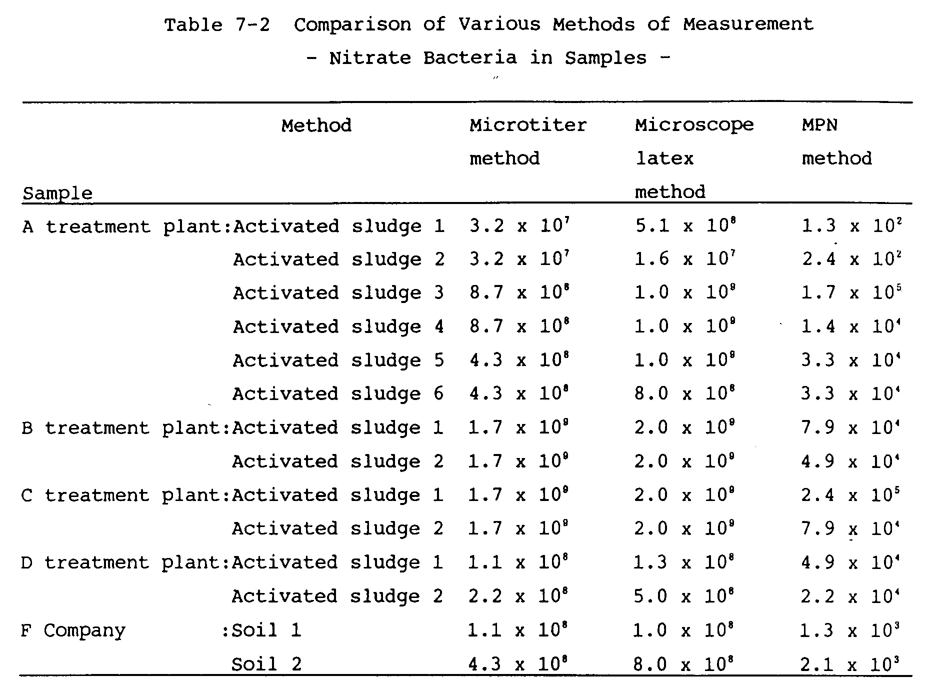

As is apparent from Table 7, in the cases of actual samples, the values obtained by the microtiter method were consistent with those obtained by the microscope latex method, but according to the MPN method, the number of bacterium measured showed small value. For it, two reasons can be considered. That is, one reason seems to be that since the MPN method is employing judgement and determination according to a coloring caused by a chemical reaction, obstruction to the coloring due to various substances included in samples occurred. The other reason seems to be that while the agglutination reaction is an antigen-antibody reaction and hence a latex aggregates dead bacteria, the value obtained by the MPN method is judged according to the results of the culture of samples and hence living bacteria alone is measured. It was confirmed, however, that a combination of the antibody-sensitized latex for detection employing latex particles with a high specific gravity of the present invention and the microtiter method of observing agglutination images with the naked eye is a preferable method for the object of this development intending to control the number of active targeted bacterium having an activity (ammonia oxidation, nitrite oxidation) being present in activated sludge and soil rapidly and properly.

### Industrial Applicability

As described in detail above, the present invention relates to an antibody-sensitized latex for detection, comprising latex particles and an antibody, wherein the antibody recognizes nitrate or nitrite bacteria specifically, and is adsorbed on the latex particles, and it becomes possible by using said antibody-sensitized latex to detect nitrate and nitrite bacteria easily and conveniently, while a conventional method for detecting them has been required a long period of time and technical expertise.

Further the antibody-sensitized latex for detection of the present invention serves to establish a method for controlling the number of targeted bacterium having an activity (ammonia oxidation, nitrite oxidation) being present in activated sludge and soil rapidly and properly.

## Claims

1. An antibody-sensitized latex used for detecting nitrate or nitrite bacteria, characterized by comprising latex particles and an antibody, wherein the antibody recognizes nitrate or nitrite bacteria specifically, and is adsorbed on the latex particles.

2. An antibody-sensitized latex used for detecting nitrate or nitrite bacteria as claimed in Claim 1, wherein said latex particles are the latex particles having a high specific gravity of about 1.5 g/cc.

3. An antibody-sensitized latex used for detecting nitrate or nitrite bacteria as claimed in Claim 1, wherein said latex particles are the latex particles having an average particle diameter of about 1.0 µm.
